(19) Europäisches Patentamt
European Patent Office
Office européen des brevets

(11) Publication number: **0 250 168**
**A1**

(12) **EUROPEAN PATENT APPLICATION**

(21) Application number: **87305195.7**

(51) Int. Cl.4: **C07C 43/13**, C07C 41/03

(22) Date of filing: **12.06.87**

(30) Priority: **19.06.86 GB 8614909**

(43) Date of publication of application:
**23.12.87 Bulletin 87/52**

(84) Designated Contracting States:
**BE DE FR GB IT NL**

(71) Applicant: **The British Petroleum Company p.l.c.**
**Britannic House Moor Lane**
**London EC2Y 9BU(GB)**

(72) Inventor: **Atkins, Martin Philip**
**The Britisch Petroleum Co. p.l.c. Chertsey Road**
**Sunbury-on-Thames Middlesex TW16 7LN(GB)**

(74) Representative: **Richardson, Derek et al**
**BP INTERNATIONAL LIMITED Patents & Agreements Division Chertsey Road**
**Sunbury-on-Thames Middlesex TW16 7LN(GB)**

(54) **Production of glycol ethers.**

(57) Glycol ethers are produced by reacting an olefin oxide with an alkanol over a cation-exchangeable lamellar clay catalyst wherein the exchangeable cations of the catalyst are cations of one or more rare earth elements.

EP 0 250 168 A1

## PRODUCTION OF GLYCOL ETHERS

This invention relates to the production of glycol ethers by reacting an olefin oxide and an alkanol over an ion-exchangeable lamellar clay catalyst.

Glycol ethers are compounds useful as jet anti-icing fluids, brake fluid blending components and solvents for paints, inks and the like. They may be produced by reacting an olefin oxide with an alkanol in the presence of either a basic or acidic catalyst.

The reaction, using ethylene oxide and propylene oxide respectively as the olefin oxide, proceeds according to the following equations:-

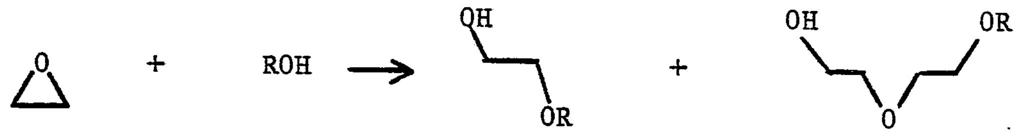

monoglycol ether diglycol ether

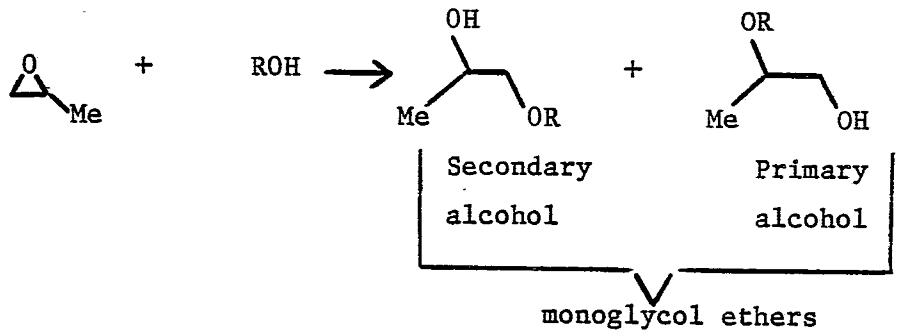

+ diglycol ethers

Secondary alcohol Primary alcohol

monoglycol ethers

Whereas with ethylene oxide the possibility of forming primary and secondary monoglycol ethers does not exist, using propylene and higher oxides both primary and secondary monoglycol ethers and higher glycol ethers may be produced, the exact proportions of which depend on the catalyst and reaction conditions employed. For example, using a base catalyst and propylene oxide as the olefin oxide, largely secondary alcohol monoglycol ethers are produced.

Thus an olefin oxide and an alkanol may be reacted in the presence of a base (e.g. sodium hydroxide) or a basic salt (e.g. potassium acetate). This homogeneous liquid phase reaction is carried out as a batch process in a stirred tank reactor at an elevated temperature starting from about 80°C and exotherming to about 160°C. The use of a basic catalyst gives a relatively low proportion of undesirable by-products, particularly the highly undesirable dioxans and substituted dioxans, but the stirred, homogeneous, batch reaction is complicated and relatively expensive, especially for product separation for which base neutralisation or wash stages are generally required. Also about 90% of the monoglycol ether product derived from propylene oxide is secondary monoglycol ether, whereas, for certain uses the primary ether is preferred.

It has been proposed to produce monoglycol ethers using an acidic catalyst, and specifically, using a lamellar clay catalyst in, for example, our European patent publication Nos. 0031687, 0031252 and 0083970. EP-A-0031687 describes the use of $H^+$-exchanged layered clays as catalysts in inter alia the production of ethers by reacting one or more epoxides with one or more alcohols, polyols or polysaccharides. EP-A-0031252 describes the use of metal cation-exchanged layered clays in a similar reaction. EP-A-0083970 describes and claims a method for carrying out a process capable of catalysis by protons using as catalyst a cation-exchangeable solid material

characterised in that

the cation-exchangeable solid material is a stabilised pillared interlayered clay and the process capable of catalysis by protons is either:

(i) a process for the production of an ether by reacting either an olefin or an olefin oxide with an alcohol, or

(ii) a process for the production of an ether by reacting either a primary or secondary aliphatic alcohol, a polyol or an olefin oxide, or

(iii) a process for the production of an alkyl aromatic compound by reacting an aromatic compound with either an alcohol or an olefin, or

(iv) a process for the production of an alcohol by reacting an olefin with water, or

(v) a process for the production of an ester by reacting either an olefin or an olefin oxide with a carboxylic acid. Claimed and exemplified are methods for reacting ethylene oxide or propylene oxide with an alkanol to produce mono-, di-, or tri-ethylene or propylene glycol mono-alkyl ethers and higher ethers.

By using a solid clay catalyst the production process is simplified since a continuous, fixed bed system can be used. However, acidic catalysts have a greater tendency than bases to produce undesirable by-products, particularly dioxan. In practice, product specifications may require the dioxan or substituted dioxan level to be below 10 ppm and not all lamellar clay catalysts are capable of achieving this low level.

It has now been found that lamellar clay catalysts can give low levels of dioxan while remaining highly active if the exchangeable cation is a rare earth metal cation.

According to the present invention a method for the production of glycol ethers by reacting an olefin oxide with an alkanol over a cation-exchangeable lamellar clay catalyst is characterised in that the exchangeable cations of the catalyst are cations of one or more rare earth elements.

For the purpose of the present invention rare earth elements are defined as elements having atomic numbers of from 57 to 71 inclusive.

The structure of lamellar clays and the mechanism by which they show activity for proton-catalysed reactions has, by now, been well studied. A good general description will be found in the afore-mentioned European Patent Publication Nos. 0083970, 0031687 and 0031252. The clays of the smectite group, which occur naturally and may also be prepared synthetically, show a lamellar structure with exchangeable cations. In nature the exchangeable cations are alkali metal and alkaline earth metal cations but these can be exchanged for hydrogen or a wide range of metal cations. The interlamellar spaces between the lamellae may be maintained by polar molecules e.g. water, cations or by pillars derived from metal hydroxides inserted into the structure.

The ion-exchangeable lamellar clay catalysts used in the present invention can be any of the smectite group clays e.g. montmorillonite, bentonite, hectorite, beidellite, and laponite. Good results have been obtained with the simple lamellar clays but pillared clays derived from smectite-type lamellar clays are preferred. Suitable pillared clays are described in, for example, US Patents Nos. 4238364; 4176090; 4271043 and 4248739. Examples of suitable pillared clays include alumina and zirconia pillared clays.

The rare earth cations may be introduced in known manner by contacting the clay with a rare earth salt solution at ambient temperature and subsequently washing with water to remove any excess solution and extraneous ions. It is important that the layered structure of the simple lamellar clays is preserved during cation-exchange. It is therefore preferred to avoid temperatures exceeding about 50°C during cation-exchange and to use drying temperatures not exceeding about 100°C. Other cation-exchange techniques may be employed if desired.

Any of the rare earth cations may be used singly or in admixture e.g. lanthanum, cerium, or a mixture of cations derived from commercially available mixtures of rare earth metal oxides known as lanthanides. Depending on the ion-exchange capacity of the clay the rare earth cation content may be from 1 to 5% wt. Preferably rare earth cations are substantially the only exchangeable cations present, i.e. there is complete rare earth ion-exchange since this gives minimum production of undesirable by-products.

As regards the reactants used, the alcohol may be a monohydric aliphatic, cycloaliphatic or aromatic alcohol. Examples of suitable aliphatic alcohols include methanol, ethanol, propanols, butanols, pentanols and hexanols. An example of a suitable cycloaliphatic alcohol is cyclohexanol and an example of an aryl alcohol is phenol. Mixtures of alcohols may be employed if desired.

With regard to the olefin oxide any suitable olefin oxide may be employed. Suitable olefin oxides include ethylene oxide and propylene oxide, or mixtures thereof. The amount of olefin or olefin oxide employed may be greater or less than the stoichiometric amount required to react completely with the alcohol. Generally, using an olefin oxide, it is preferred to employ an excess of the alcohol in order to maximise the yield of desired ether. Preferably the excess of alcohol to olefin oxide is from 2:1 to 20:1 (molar), lower ratios within this range tending to produce larger amounts of di-and higher ethers.

Monoglycol ethers e.g. monoethylene glycol mono-methyl, -ethyl or -butyl ethers may be produced by reacting ethylene oxide with methanol, ethanol or butanol respectively; mono-propylene glycol monoalkyl ethers may be produced by reacting propylene oxide with an alkanol. There may be some production of di-, tri-or poly-glycol ethers, the relative proportions of which may be altered by choice of process conditions. Suitable preliminary experiments can be used to optimise the reactor conditions for any given feedstock and catalyst.

The process may be carried out in the liquid phase or the vapour phase, but preferably the former. Suitable ranges of temperature and pressure may be:-

| | | Broad Range | Preferred Range |
|---|---|---|---|
| Temperature | °C | 0 to 180 | 70 to 100 |
| Pressure | bars gauge | 1 to 100 | 15 to 50 |

The process may be carried out continuously or batchwise, preferably the former. For continuous operation the other main process variable is the liquid hourly space velocity which may be as follows:-

| | | Broad Range | Preferred Range |
|---|---|---|---|
| LHSV | $v/v/hr^{-1}$ | 0.5 to 10 | 1 to 5 |

By a suitable choice of process conditions within the above ranges it has been found possible to keep the production of di-and higher-glycol ethers to below 40% wt of the glycol ether product and the production of dioxan or substituted dioxans to below 5 ppm by wt of the total reaction product. (This being the normal limit of detectability by gas chromatography/mass spectroscopy (GCMS)) The following Examples illustrate the preparation and use of rare earth cation-exchanged catalysts.

In the Example a rare earth cations solution was prepared from a mixture of rare earth metals supplied by Johnson Matthey. The solution contained ca. 250g/l $Ln_2O_3$ and was cerium free. Its detailed composition determined by XRF analysis and based on the oxides was:-

| Metal | % |
|---|---|
| La | 51.0 |
| Nd | 37.5 |
| Pr | 7.0 |
| Sm | 2.8 |
| Y | 0.1 |
| Gd | 1.1 |
| Er | 0.4 |
| Others | less than 0.1 |

Preparation of Pillared Clays

Wyoming montmorillonite clay (1143 g) was added to a stirred suspension of CHLORHYDROL (RTM) (1143 g) in water (20 litre). The pH of the solution was adjusted to pH 5.5 with aqueous ammonia and maintained at this pH while the mixture was heated for 1h at 65°C. The pillared interlayered clay obtained was separated by decantation. It was washed with deionised water, dried at 80-100°C and calcined in air at 450-500°C.

The silicon:aluminium ratio of the product was 1.3:1 by weight and the basal $d_{001}$ spacing was 17.8 Angstrom.

Cation-exchange of Pillared Clay

20 g of alumina pillared clay was added to 200 $cm^3$ of a 0.5 M solution of rare earth nitrates at room temperature and left for 1 hour. The clay was filtered and washed with distilled water to remove extraneous ions.

This ion-exchange process was repeated twice more after which the ion-exchanged clay was dried in an oven at 100°C for 6 hours and calcined in air at 450°C for 4 hours.

The rare earth cation content of the clay was circa 3 % wt.

The method of the invention is illustrated by the following Example.

Example

15 g of rare earth ion-exchanged clay of Example A were placed in a continuous flow reactor and used to react propylene oxide and ethanol under the following liquid phase process conditions:-

| | | |
|---|---|---|
| Temperature | °C | 80 |
| Pressure | bars gauge | 30 |
| LHSV | $v/v/hr^{-1}$ | 2 |
| Propylene oxide/ ethanol mole ratio | | 1:10 |

The run was continued for 8 hours and the products were analysed by GC.

There was quantitative conversion of the propylene oxide. The analysis of the reaction product was as follows:-

Primary mono-propylene glycol-monoethyl ether 5.86% wt

Secondary mono-propylene glycol-monoethyl ether 5.08% wt

Ratio of primary to secondary ether 1.2:1

Higher boiling ethers 4.15% wt

Dioxan not detectable (below 5 ppm)

Comparative Examples

Using the same process conditions except that ethylene oxide/ethanol and an LHSV of $4h^{-1}$ were used and clays ion-exchanged with other metals, the following results were obtained:-

| | Ethylene oxide Conversion | Dioxan Production |
|---|---|---|
| Mn-exchanged pillared montmorillonite | 70 | 60 ppm |
| Cs-exchanged pillared montmorillonite | Less than 5% conversion | - |
| Al-exchanged pillared montmorillonite | 100 | 1000 ppm |

**Claims**

1 A method for the production of glycol ethers by reacting an olefin oxide with an alkanol over a cation-exchangeable lamellar clay catalyst

characterised in that

the exchangeable cations of the catalyst are cations of one or more rare earth elements.

2 A method according to claim 1 wherein the lamellar clay is a smectite-type clay.

3 A method according to claim 1 wherein the lamellar clay is a pillared clay derived from a smectite-type lamellar clay.

4 A method according to any one of the preceding claims wherein the rare earth cation is either lanthanum or cerium.

5 A method according to any one of claims 1 to 3 wherein the exchangeable cations of the catalyst are a mixture of rare earth cations.

6 A method according to any one of the preceding claims wherein the catalyst is fully cation-exchanged with rare earth cations.

7 A method according to any one of the preceding claims wherein an excess of the alcohol is employed.

8 A method according to any one of the preceding claims wherein ethylene oxide is reacted with either methanol, ethanol or butanol to produce the corresponding monoethylene glycol mono-methyl, -ethyl or -butyl ether.

9 A method according to any one of claims 1 to 7 wherein propylene oxide is reacted with an alkanol to produce a monopropylene glycol monoalkyl ether.

10 A method according to any one of the preceding claims when operated in the liquid phase.

European Patent Office

# EUROPEAN SEARCH REPORT

Application number

EP 87 30 5195

| Category | DOCUMENTS CONSIDERED TO BE RELEVANT<br>Citation of document with indication, where appropriate, of relevant passages | Relevant to claim | CLASSIFICATION OF THE APPLICATION (Int. Cl.4) |
|---|---|---|---|
| A | GB-A-2 035 306 (SHOWA DENKO)<br>* Claims; examples *<br>--- | 1-10 | C 07 C 43/13<br>C 07 C 41/03 |
| A,D | EP-A-0 031 252 (BRITISH PETROLEUM)<br>* Claims 1-3,28; example 37 *<br>----- | 1-10 | |
| | | | TECHNICAL FIELDS SEARCHED (Int. Cl.4) |
| | | | C 07 C 41/00<br>C 07 C 43/00 |

The present search report has been drawn up for all claims

| Place of search | Date of completion of the search | Examiner |
|---|---|---|
| THE HAGUE | 24-09-1987 | WRIGHT M.W. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03.82